# EUROPEAN PATENT SPECIFICATION

(11) **EP 1 551 409 B1**
(45) Date of publication and mention of the grant of the patent: **21.03.2007**
(21) Application number: 03751032.8
(22) Date of filing: 06.10.2003
(51) Int. Cl.: A61K 31/517, A61K 31/7068, A61P 35/00

(54) **USE OF THE QUINAZOLINE DERIVATIVE ZD6474 COMBINED WITH GEMCITABINE AND OPTIONALLY IONISING RADIATION IN THE TREATMENT OF CANCER**
VERWENDUNG VON DEM QUINAZOLIN DERIVAT ZD6474 KOMBINIERT MIT GEMCITABIN UND GEGEBENENFALLS IONISIERENDER STRAHLUNG FÜR DIE BEHANDLUNG VON KREBS
UTILISATION DU DERIVE DE LA QUINAZOLINE ZD6474 COMBINE A LA GEMCITABINE ET EVENTUELLEMENT AU RAYONNEMENT IONISANT DANS LE TRAITEMENT DU CANCER

(30) Priority: 09.10.2002 GB 0223380
(43) Date of publication of application: 13.07.2005
(73) Proprietor: AstraZeneca AB, 151 85 Södertälje (SE)
(72) Inventor: BARGE, Alan, AstraZeneca R & D Alderley, Macclesfield, Cheshire SK10 4TG (GB)
(86) International application number: PCT/GB2003/004334
(87) International publication number: WO 2004/032937

(56) References cited:
- WO-A-01/32651
- CIARDIELLO F ET AL: "Antitumor effects of ZD6474, a small molecule VEGF receptor tyrosine kinase inhibitor that is also active against EGF receptor tyrosine kinase" PROCEEDINGS OF THE AMERICAN ASSOCIATION FOR CANCER RESEARCH ANNUAL, vol. 43, March 2002 (2002-03), pages 1080-1081, XP001157108 93rd Annual Meeting of the American Association for Cancer Research;San Francisco, California, USA; April 06-10, 2002, March, 2002 ISSN: 0197-016X
- DREVS J ET AL: "Effect of ZD6474, a VEGF receptor tyrosine kinase inhibitor, on primary tumor growth, metastasis, vessel density and microvascular architecture in murine renal cell carcinoma" PROCEEDINGS OF THE AMERICAN ASSOCIATION FOR CANCER RESEARCH ANNUAL, vol. 43, March 2002 (2002-03), page 1082 XP001156998 93rd Annual Meeting of the American Association for Cancer Research;San Francisco, California, USA; April 06-10, 2002, March, 2002 ISSN: 0197-016X
- WEDGE S R ET AL: "Combination of the VEGF receptor tyrosine kinase inhibitor ZD6474 and vascular-targeting agent ZD6126 produces an enhanced antitumor response" PROCEEDINGS OF THE AMERICAN ASSOCIATION FOR CANCER RESEARCH ANNUAL, vol. 43, March 2002 (2002-03), page 1081 XP001156999 93rd Annual Meeting of the American Association for Cancer Research;San Francisco, California, USA; April 06-10, 2002, March, 2002 ISSN: 0197-016X
- HOLDEN S ET AL: "Effects of ZD6474, an orally active inhibitor of VEGF receptor tyrosine kinase, in patients with solid tumors: Results from a phase I study" EUROPEAN JOURNAL OF CANCER, PERGAMON PRESS, OXFORD, GB, vol. 37, no. Supplement 6, October 2001 (2001-10), page S73 XP002256123 ISSN: 0959-8049
- HENNEQUIN L F ET AL: "Novel 4-anilinoquinazolines with C-7 basic side chains: Design and structure activity relationship of a series of potent, orally active, VEGF receptor tyrosine kinase inhibitors" JOURNAL OF MEDICINAL CHEMISTRY, AMERICAN CHEMICAL SOCIETY. WASHINGTON, US, vol. 45, no. 6, 14 March 2002 (2002-03-14), pages 1300-1312, XP002256124 ISSN: 0022-2623
- GIACCONE G ET AL: "ZD1839 ('Iressa'), an orally-active, selective, epidermal growth factor receptor tyrosine kinase inhibitor (egfr-tki), is well tolerated in combination with gemcitabine and cisplatin, in patients with advanced solid tumours: Preliminary tolerability, efficacy and pharmacokinetic results" EUROPEAN JOURNAL OF CANCER, vol. 37, no. Supplement 6, October 2001 (2001-10), pages S30-S31, XP002266469 11th European Cancer Conference;Lisbon, Portugal; October 21-25, 2001 ISSN: 0959-8049

## Description

The present invention relates to a pharmaceutical composition comprising ZD6474 and gemcitabine; to a kit comprising ZD6474 and gemcitabine; to the use of ZD6474 and gemcitabine in the manufacture of a medicament for use in the treatment of a cancer in a warm-blooded animal such as a human which is optionally being treated with ionising radiation.

Normal angiogenesis plays an important role in a variety of processes including embryonic development, wound healing and several components of female reproductive function. Undesirable or pathological angiogenesis has been associated with disease states including diabetic retinopathy, psoriasis, cancer, rheumatoid arthritis, atheroma, Kaposi's sarcoma and haemangioma (Fan et al, 1995, Trends Pharmacol. Sci. 16: 57-66; Folkman, 1995, Nature Medicine 1: 27-31). Alteration of vascular permeability is thought to play a role in both normal and pathological physiological processes (Cullinan-Bove et al, 1993, Endocrinology 133: 829-837; Senger et al, 1993, Cancer and Metastasis Reviews, 12: 303-324). Several polypeptides with *in vitro* endothelial cell growth promoting activity have been identified including, acidic and basic fibroblast growth factors (aFGF & bFGF) and vascular endothelial growth factor (VEGF). By virtue of the restricted expression of its receptors, the growth factor activity of VEGF, in contrast to that of the FGFs, is relatively specific towards endothelial cells. Recent evidence indicates that VEGF is an important stimulator of both normal and pathological angiogenesis (Jakeman et al, 1993, Endocrinology, 133: 848-859; Kolch et al, 1995, Breast Cancer Research and Treatment, 36:139-155) and vascular permeability (Connolly et al, 1989, J. Biol. Chem. 264: 20017-20024). Antagonism of VEGF action by sequestration of VEGF with antibody can result in inhibition of tumour growth (Kim et al, 1993, Nature 362: 841-844).

Receptor tyrosine kinases (RTKs) are important in the transmission of biochemical signals across the plasma membrane of cells. These transmembrane molecules characteristically consist of an extracellular ligand-binding domain connected through a segment in the plasma membrane to an intracellular tyrosine kinase domain. Binding of ligand to the receptor results in stimulation of the receptor-associated tyrosine kinase activity which leads to phosphorylation of tyrosine residues on both the receptor and other intracellular molecules. These changes in tyrosine phosphorylation initiate a signalling cascade leading to a variety of cellular responses. To date, at least nineteen distinct RTK subfamilies, defined by amino acid sequence homology, have been identified. One of these subfamilies is presently comprised by the fms-like tyrosine kinase receptor, Flt-1, the kinase insert domain-containing receptor, KDR (also referred to as Flk-I), and another fms-Iike tyrosine kinase receptor, Flt-4. Two of these related RTKs, Flt-1 and KDR, have been shown to bind VEGF with high affinity (De Vries et al, 1992, Science 255: 989-991; Terman et al, 1992, Biochem Biophys. Res. Comm 1992, 187: 1579-1586). Binding of VEGF to these receptors expressed in heterologous cells has been associated with changes in the tyrosine phosphorylation status of cellular proteins and calcium fluxes.

Quinazoline derivatives which are inhibitors of VEGF receptor tyrosine kinase are described in International Patent Applications Publication Nos. WO 98/13354 and WO 01/32651. In WO 98/13354 and WO 01/32651 compounds are described which possess activity against VEGF receptor tyrosine kinase (VEGF RTK) whilst possessing some activity against epidermal growth factor (EGF) receptor tyrosine kinase (EGF RTK). ZD6474 is 4-(4-bromo-2-fluoroanilino)-6-methoxy-7-(1-methylpiperidin-4-ylmethoxy)quinazoline:

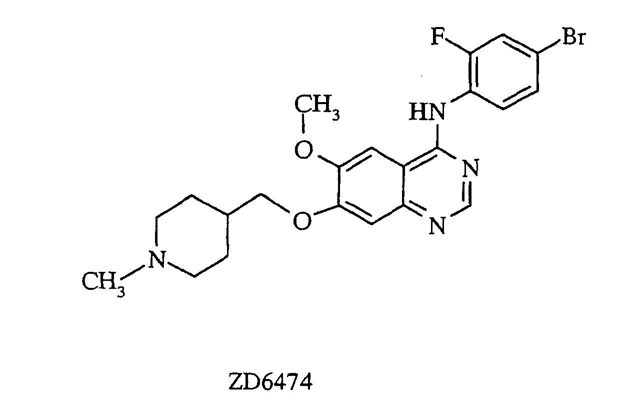

ZD6474 falls within the broad general disclosure of WO 98/13354 and is exemplified in WO 01/32651. ZD6474 is a potent inhibitor of VEGF RTK and also has some activity against EGF RTK. ZD6474 has been shown to elicit broad-spectrum anti-tumour activity in a range of models following once-daily oral administration (Wedge S.R., Ogilvie D.J., Dukes M. et al, Proc. Am. Assoc. Canc. Res. 2001; 42: abstract 3126).

In WO 98/13354 and WO 01/32651 it is stated that compounds of their inventions: "may be applied as a sole therapy or may involve, in addition to a compound of the invention, one or more other substances and/or treatments. Such conjoint treatment may be achieved by way of the simultaneous, sequential or separate administration of the individual components of the treatment."
WO 98/13354 and WO 01/32651 then go on to describe examples of such conjoint treatment including surgery, radiotherapy and various types of chemotherapeutic agent including inhibitors of growth factor function.

Nowhere in WO 98/13354 and WO 01/32651 does it suggest the combination of a compound of the invention and gemcitabine for the treatment of any disease state including cancer.

Nowhere in WO 98/13354 and WO 01/32651 is the specific combination ofZD6474 and gemcitabine suggested.

Nowhere in WO 98/13354 and WO 01/32651 does it state that use of any compound of the invention therein with other treatments will produce surprisingly beneficial effects.

A triple combination of a VEGF RTK inhibitor (PTK 787), an EGF RTK inhibitor (PKI 166) and gemcitabine is described in Baker et al, Cancer Research 62, 1996 2003, April 1, 2002. The authors concluded that a combination of either PTK 787 with gemcitabine or PKI 166 with gemcitabine was beneficial but that the triple combination did not produce additive therapeutic effects.

A combination of gemcitabine with DC101, a VEGF receptor-2 antibody (anti-KDR antibody) is described in Bruns et al, International Journal of Cancer vol 102, issue 2, 2002 pages 101-108.

The abstract by Ciardiello F. et al, Proc. Am. Assoc. Canc. Res. Ann. vol 43, March 2002, (2002-03) pages 1080-1081, describes the inhibition of epidermal growth factor receptor (EGFR) by ZD6474 in NIH-3T3 mouse fibroblasts that over express the human EGFR and in human MCF-10A ras breast cancer cells. It goes on to describe the treatment of nude mice with GEO colon cancer xenografts with a combination of ZD6474 and paclitaxel. Drevs J. et al, Proc. Am. Assoc. Canc. Res. Ann. vol 43, March 2002, (2002-03) page 1082, describes the use of magnetic resonance imaging (MRI) to look at vascular parameters like vessel density and microvascular architecture in a model of murine renal cell carcinoma. Wedge S. R. et al, Proc. Am. Assoc. Canc. Res. Ann. vol 43, March 2002, (2002-03) page 1081, describes the combination of ZD6474 and ZD6126 (N-acetylcolchinol-O-phosphate) in a Calu-6 human lung tumour xenograft model and in a LoVo human colorectal tumour xenograft model.
Holden S. et al, Eur. Jnl. Canc. vol 37, no. supplement 6, 2001, page S73, gives a preliminary review of a Phase I clinical trial of ZD6474 in patients with solid tumours.
Hennequin L. F et al, Jnl. Med. Chem. 45, 6, 2002, 1300-1312, describes the structure activity relationship of a series of VEGF RTK inhibitors that includes ZD6474.

Unexpectedly and surprisingly we have now found that the particular compound ZD6474 used in combination with a particular selection from the broad description of combination therapies listed in WO 98/13354 and WO O1/32651, namely with gemcitabine, produces significantly better effects than any one of ZD6474 and gemcitabine used alone. In particular, ZD6474 used in combination with gemcitabine produces significantly better effects on solid tumours than any one of ZD6474 and gemcitabine used alone.

Gemcitabine is (INN) 2'-deoxy-2',2'-difluorocytidine monohydrochloride (β-isomer).

Gemcitabine is also known as Gemzar^{™} (Trademark of Lilly) and it is a cytotoxic agent. It is an anti-metabolite which causes inhibition of DNA synthesis.

Anti-cancer effects of the present invention include anti-tumour effects, the response rate, the time to disease progression and the survival rate. Anti-tumour effects of the present invention include inhibition of tumour growth, tumour growth delay, regression of tumour, shrinkage of tumour, increased time to regrowth of tumour on cessation of treatment, slowing of disease progression. It is expected that when a pharmaceutical composition of the present invention is administered to a warm-blooded animal such as a human, in need of treatment for cancer, with or without a solid tumour, said treatment will produce an effect, as measured by, for example, one or more of: the extent of the anti-tumour effect, the response rate, the time to disease progression and the survival rate.

According to the present invention there is provided a pharmaceutical composition which comprises ZD6474 or a pharmaceutically acceptable salt thereof, and gemcitabine, in association with a pharmaceutically acceptable excipient or carrier.

According to a further aspect of the present invention there is provided a combination product comprising ZD6474 or a pharmaceutically acceptable salt thereof and gemcitabine, for use in a method of treatment of a human or animal body by therapy.

According to a further aspect of the present invention there is provided a kit comprising ZD6474 or a pharmaceutically acceptable salt thereof, and gemcitabine.

According to a further aspect of the present invention there is provided a kit comprising:
a) ZD6474 or a pharmaceutically acceptable salt thereof in a first unit dosage form;
b) gemcitabine in a second unit dosage form; and
c) container means for containing said first and second dosage forms.

According to a further aspect of the present invention there is provided a kit comprising:
a) ZD6474 or a pharmaceutically acceptable salt thereof, together with a pharmaceutically acceptable excipient or carrier, in a first unit dosage form;
b) gemcitabine together with a pharmaceutically acceptable excipient or carrier, in a second unit dosage form; and
c) container means for containing said first and second dosage forms.

According to a further aspect of the present invention there is provided the use of ZD6474 or a pharmaceutically acceptable salt thereof and gemcitabine in the manufacture of a medicament for use in the treatment of a cancer in a warm-blooded animal such as a human.

According to a further aspect of the present invention there is provided the use of ZD6474 or a pharmaceutically acceptable salt thereof and gemcitabine in the manufacture of a medicament for use in the treatment of a cancer involving a solid tumour in a warm-blooded animal such as a human.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the simultaneous, sequential or separate administration of an effective amount of gemcitabine, wherein gemcitabine may optionally be administered together with a pharmaceutically acceptable excipient or carrier, to a warm-blooded animal such as a human in need of such therapeutic treatment.
Such therapeutic treatment includes an antiangiogenic and/or vascular permeability effect, an anti-cancer effect and an anti-tumour effect.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic agent in addition to a combination treatment of the invention.

Surgery may comprise the step of partial or complete tumour resection, prior to, during or after the administration of the combination treatment with ZD6474 described herein.

Other chemotherapeutic agents for optional use with a combination treatment of the present invention include those described in WO 01/32651. Such chemotherapy may cover five main categories of therapeutic agent:
(i) other antiangiogenic agents including vascular targeting agents;
(ii) cytostatic agents;
(iii) biological response modifiers (for example interferon);
(iv) antibodies (for example edrecolomab); and
(v) antiproliferative/antineoplastic drugs and combinations thereof, as used in medical oncology.
A particular example of a chemotherapeutic agent for use with a combination treatment of the present invention is a platinum anti-cancer agent such as cisplatin; such a combination is expected to be particularly useful for the treatment of lung cancer and bladder cancer.

The administration of a triple combination of ZD6474, gemcitabine and ionising radiation may produce effects, such as anti-tumour effects, greater than those achieved with any of ZD6474, gemcitabine and ionising radiation used alone, greater than those achieved with the combination of ZD6474 and gemcitabine, greater than those achieved with the combination of ZD6474 and ionising radiation, greater than those achieved with the combination of gemcitabine and ionising radiation.

According to a further aspect of the present invention there is provided the use of ZD6474 or a pharmaceutically acceptable salt thereof and gemcitabine in the manufacture of a medicament for use for use in the treatment of a cancer in a warm-blooded animal such as a human which is being treated with ionising radiation.

According to a further aspect of the present invention there is provided the use of ZD6474 or a pharmaceutically acceptable salt thereof and gemcitabine in the manufacture of a medicament for use in the treatment of a cancer involving a solid tumour in a warm-blooded animal such as a human which is being treated with ionising radiation.

According to a further aspect of the present invention there is provided a therapeutic combination treatment comprising the administration of an effective amount of ZD6474 or a pharmaceutically acceptable salt thereof, optionally together with a pharmaceutically acceptable excipient or carrier, and the administration of an effective amount of gemcitabine, optionally together with a pharmaceutically acceptable excipient or carrier and the administration of an effective amount of ionising radiation, to a warm-blooded animal such as a human in need of such therapeutic treatment wherein the ZD6474, gemcitabine and ionising radiation may be administered simultaneously, sequentially or separately and in any order.

A warm-blooded animal such as a human which is being treated with ionising radiation means a warm-blooded animal such as a human which is treated with ionising radiation before, after or at the same time as the administration of a medicament or combination treatment comprising ZD6474 and gemcitabine. For example said ionising radiation may be given to said warm-blooded animal such as a human within the period of a week before to a week after the administration of a medicament or combination treatment comprising ZD6474 and gemcitabine. This means that ZD6474, gemcitabine and ionising radiation may be administered separately or sequentially in any order, or may be administered simultaneously. The warm-blooded animal may experience the effect of each of ZD6474, gemcitabine and radiation simultaneously.

According to one aspect of the present invention the ionising radiation is administered before one of ZD6474 and gemcitabine or after one of ZD6474 and gemcitabine.

According to one aspect of the present invention the ionising radiation is administered before both ZD6474 and gemcitabine or after both ZD6474 and gemcitabine.

According to one aspect of the present invention ZD6474 is administered to a warm-blooded animal after the animal has been treated with ionising radiation.

According to another aspect of the present invention the effect of treatment of the present invention is expected to be at least equivalent to the addition of the effects of each of the components of said treatment used alone, that is, of each of ZD6474 and gemcitabine used alone or of each of ZD6474, gemcitabine and ionising radiation used alone.

According to another aspect of the present invention the effect of treatment of the present invention is expected to be greater than the addition of the effects of each of the components of said treatment used alone, that is, of each of ZD6474 and gemcitabine used alone or of each of ZD6474, gemcitabine and ionising radiation used alone.

According to another aspect of the present invention the effect of treatment of the present invention is expected to be a synergistic effect.

According to the present invention a combination treatment is defined as affording a synergistic effect if the effect is therapeutically superior, as measured by, for example, the extent of the response, the response rate, the time to disease progression or the survival period, to that achievable on dosing one or other of the components of the combination treatment at its conventional dose. For example, the effect of the combination treatment is synergistic if the effect is therapeutically superior to the effect achievable with ZD6474 or gemcitabine or ionising radiation alone. Further, the effect of the combination treatment is synergistic if a beneficial effect is obtained in a group of patients that does not respond (or responds poorly) to ZD6474 or gemcitabine or ionising radiation alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the response rate, the time to disease progression or the survival period, is equivalent to that achievable on dosing conventional amounts of the components of the combination treatment. In particular, synergy is deemed to be present if the conventional dose of ZD6474 or gemcitabine or ionising radiation may be reduced without detriment to one or more of the extent of the response, the response rate, the time to disease progression and survival data, in particular without detriment to the duration of the response, but with fewer and/or less troublesome side-effects than those that occur when conventional doses of each component are used.

As stated above the combination treatments of the present invention as defined herein are of interest for their antiangiogenic and/or vascular permeability effects. Angiogenesis and/or an increase in vascular permeability is present in a wide range of disease states including cancer (including leukaemia, multiple myeloma and lymphoma), diabetes, psoriasis, rheumatoid arthritis, Kaposi's sarcoma, haemangioma, acute and chronic nephropathies, atheroma, arterial restenosis, autoimmune diseases, acute inflammation, lymphoedema, endometriosis, dysfunctional uterine bleeding and ocular diseases with retinal vessel proliferation including age-related macular degeneration. Combination treatments of the present invention are expected to be particularly useful in the prophylaxis and treatment of diseases such as cancer and Kaposi's sarcoma. In particular such combination treatments of the invention are expected to slow advantageously the growth of primary and recurrent solid tumours of, for example, the colon, pancreas, bladder, breast, prostate, lungs and skin. More especially combination treatments of the present invention are expected to slow advantageously the growth of tumours in pancreatic cancer and lung cancer, for example mesothelioma and non-small cell lung cancer (NSCLC). More particularly such combination treatments of the invention are expected to inhibit any form of cancer associated with VEGF including leukaemia, mulitple myeloma and lymphoma and also, for example, to inhibit the growth of those primary and recurrent solid tumours which are associated with VEGF, especially those tumours which are significantly dependent on VEGF for their growth and spread, including for example, certain tumours of the colon, pancreas, bladder, breast, prostate, lung, vulva and skin, particularly NSCLC.

In another aspect of the present invention ZD6474 and gemcitabine, optionally with ionising radiation, are expected to inhibit the growth of those primary and recurrent solid tumours which are associated with VEGF especially those tumours which are significantly dependent on VEGF for their growth and spread.

In another aspect of the present invention ZD6474 and gemcitabine, optionally with ionising radiation, are expected to inhibit the growth of those primary and recurrent solid tumours which are associated with both VEGF and EGF especially those tumours which are significantly dependent on VEGF and EGF for their growth and spread.

The compositions described herein may be in a form suitable for oral administration, for example as a tablet or capsule, for nasal administration or administration by inhalation, for example as a powder or solution, for parenteral injection (including intravenous, subcutaneous, intramuscular, intravascular or infusion) for example as a sterile solution, suspension or emulsion, for topical administration for example as an ointment or cream, for rectal administration for example as a suppository or the route of administration may be by direct injection into the tumour or by regional delivery or by local delivery. In other embodiments of the present invention the ZD6474 of the combination treatment may be delivered endoscopically, intratracheally, intralesionally, percutaneously, intravenously, subcutaneously, intraperitoneally or intratumourally. Preferably ZD6474 is administered orally. In general the compositions described herein may be prepared in a conventional manner using conventional excipients. The compositions of the present invention are advantageously presented in unit dosage form

ZD6474 will normally be administered to a warm-blooded animal at a unit dose within the range 10-500nig per square metre body area of the animal, for example approximately 0.3-15mg/kg in a human. A unit dose in the range, for example, 0.3-15mg/kg, preferably 0.5-5mg/kg is envisaged and this is normally a therapeutically-effective dose. A unit dosage form such as a tablet or capsule will usually contain, for example 25-500mg of active ingredient. Preferably a daily dose in the range of 0.5-5mg/kg is employed.

Gemcitabine may be administered according to known clinical practice. For example in NSCLC the recommended dose of gemcitabine is 1000mg/m² given by 30 minute intravenous infusion. This may be repeated once weekly for three weeks, followed by a one week rest period. This four week cycle may then be repeated. Dosage reduction may be necessary if the patient experiences undue toxicity. In pancreatic cancer the recommended dose of gemcitabine is 1000mg/m² given by 30 minute intravenous infusion. This may be repeated once weekly for seven weeks followed by a week of rest. Subsequent cycles may consist of injections once weekly for three consecutive weeks out of every four weeks. Dosage reduction may be necessary if the patient experiences undue toxicity.
The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of γ-rays, X-rays, and/or the directed delivery of radiation from radioisotopes. Other forms of DNA damaging factors are also included in the present invention such as microwaves and UV-irradiation. For example X-rays may be dosed in daily doses of 1.8-2.0Gy, 5 days a week for 5-6 weeks. Normally a total fractionated dose will lie in the range 45-60Gy. Single larger doses, for example 5-10Gy may be administered as part of a course of radiotherapy. Single doses may be administered intraoperatively. Hyperfractionated radiotherapy may be used whereby small doses of X-rays are administered regularly over a period of time, for example 0.1Gy per hour over a number of days. Dosage ranges for radioisotopes vary widely, and depend on the half-life of the isotope, the strength and type of radiation emitted, and on the uptake by cells.

As stated above the size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

The present invention relates to combinations of gemcitabine with ZD6474 or with a salt of ZD6474.

Salts of ZD6474 for use in pharmaceutical compositions will be pharmaceutically acceptable salts, but other salts may be useful in the production of ZD6474 and its pharmaceutically acceptable salts. Such salts may be formed with an inorganic or organic base which affords a pharmaceutically acceptable cation. Such salts with inorganic or organic bases include for example an alkali metal salt, such as a sodium or potassium salt, an alkaline earth metal salt such as a calcium or magnesium salt, an ammonium salt or for example a salt with methylamine, dimethylamine, trimethylamine, piperidine, morpholine or tris-(2-hydroxyethyl)amine.

ZD6474 may be synthesised according to any of the known processes for making ZD6474. For example ZD6474 may be made according to any of the processes described in WO 01/32651; for example those described in Examples 2(a), 2(b) and 2(c) of WO 01/32651.

Gemcitabine is commercially available.

The following tests may be used to demonstrate the activity of ZD6474 in combination with gemcitabine.

### Model to investigate effects of ZD6474 and gemcitabine therapy on primary pancreatic tumour growth and metastases growing orthotopically in nude mice

Nude mice (n=10 per group) were injected with 1x10⁶ L3.6pl human pancreatic cancer cells into the pancreas. The mice received one of the following treatment regimes 8 days post-tumour injection:
Group 1 - control group received saline by daily oral application;
Group 2 - a single daily dose of ZD6474 (50mg/kg oral gavage);
Group 3 - gemcitabine twice weekly (on days 2 and 5 of each treatment week) (100mg/kg by intraperitoneal injection (i.p.));
Group 4 - a single daily dose of ZD6474 (50mg/kg oral gavage) and twice weekly gemcitabine (on days 2 and 5 of each treatment week) (GEM: 100mg/kg i.p.).
The animals were sacrificed 32 days after tumour cell injection.
The incidence of pancreatic tumours, liver metastases, lymph node metastases and peritoneal carcinosis was evaluated. The primary pancreatic tumour weight was measured. All macroscopically enlarged lymph nodes and liver nodules were confirmed by histopathology (H&E staining).
The results are shown in Table 1

**Table 1**

| Therapy Group | Incidence Pancreas | Liver Met | LN Met | Peritoneal Carcinosis | Tumour Weight Average +/- std dev | Body Weight Average +/- std dev |
|---|---|---|---|---|---|---|
| Control | 10/10 | 6/10 | 10/10 | 3/10 | mg 1231 +/- 290 | g 21.4 +/- 2.2 |
| ZD6474 | 10/10 | 1/10 | 3/10 | 1/10 | 541 +/- 201 | 19.2 +/- 2.9 |
| Gemcitabine | 9/9 | 4/9 | 9/9 | 3/9 | 836 +/- 291 | 21.7 +/- 2.1 |
| ZD6474 + Gemcitabine | 5/5 | 0/5 | 1/5 | 0/5 | 308 +/- 129 | 19.3 +/- 3.4 |

Compared with the average weight of control tumours (1231mg), tumours in treated animals reached an average weight of 836mg (gemcitabine) 541mg (ZD6474) and 308mg (gemcitabine + ZD6474). Liver metastases were present in 6/10 control and 4/9 gemcitabine treated animals, but only 1/10 and 0/5 animals on ZD6474 or combination treatment displayed liver metastases, respectively. Lymph node metastases were present in 10/10 control and 9/9 gemcitabine treated animals, but only 3/10 and 1/5 animals on ZD6474 or combination treatment displayed lymph node metastases, respectively.

## Claims

1. Use of ZD6474 or a pharmaceutically acceptable salt thereof and gemcitabine in the manufacture of a medicament for use in the treatment of a cancer in a warm-blooded animal such as a human.

2. Use according to claim 1 wherein the cancer involves a solid tumour.

3. Use according to claim 1 wherein the cancer is pancreatic cancer.

4. Use according to claim 1 wherein the cancer is lung cancer.

5. Use of ZD6474 or a pharmaceutically acceptable salt thereof and gemcitabine in the manufacture of a medicament for use in the treatment of a cancer in a warm-blooded animal such as a human which is being treated with ionising radiation.

6. Use according to claim 5 wherein the cancer involves a solid tumour.

7. Use according to claim 5 wherein the cancer is pancreatic cancer.

8. Use according to claim 5 wherein the cancer is lung cancer.

9. A pharmaceutical composition which comprises ZD6474 or a pharmaceutically acceptable salt thereof, and gemcitabine, in association with a pharmaceutically acceptable excipient or carrier.

10. A kit comprising ZD6474 or a pharmaceutically acceptable salt thereof, and gemcitabine.

## Patentansprüche

1. Verwendung von ZD6474 oder einem pharmazeutisch annehmbaren Salz davon und Gemcitabin bei der Herstellung eines Arzneimittels zur Verwendung bei der Behandlung einer Krebserkrankung bei einem Warmblüter wie dem Menschen.

2. Verwendung nach Anspruch 1, bei der bei der Krebserkrankung ein solider Tumor vorliegt.

3. Verwendung nach Anspruch 1, bei der es sich bei der Krebserkrankung um Pankreaskrebs handelt.

4. Verwendung nach Anspruch 1, bei der es sich bei der Krebserkrankung um Lungenkrebs handelt.

5. Verwendung von ZD6474 oder einem pharmazeutisch annehmbaren Salz davon und Gemcitabin bei der Herstellung eines Arzneimittels zur Verwendung bei der Behandlung einer Krebserkrankung bei einem Warmblüter wie dem Menschen, der mit ionisierender Strahlung behandelt wird.

6. Verwendung nach Anspruch 5, bei der bei der Krebserkrankung ein solider Tumor vorliegt.

7. Verwendung nach Anspruch 5, bei der es sich bei der Krebserkrankung um Pankreaskrebs handelt.

8. Verwendung nach Anspruch 5, bei der es sich bei der Krebserkrankung um Lungenkrebs handelt.

9. Pharmazeutische Zusammensetzung, enthaltend ZD6474 oder ein pharmazeutisch annehmbares Salz davon und Gemcitabin zusammen mit einem pharmazeutisch annehmbaren Hilfsstoff oder Träger.

10. Kit, enthaltend ZD6474 oder ein pharmazeutisch annehmbares Salz davon und Gemcitabin.

## Revendications

1. Utilisation du ZD6474 ou d'un sel pharmaceutiquement acceptable de celui-ci et de la gemcitabine dans la fabrication d'un médicament destiné à être utilisé dans le traitement d'un cancer chez un animal à sang chaud tel que l'homme.

2. Utilisation selon la revendication 1, **caractérisée en ce que** le cancer implique une tumeur solide.

3. Utilisation selon la revendication 1, **caractérisée en ce que** le cancer est le cancer pancréatique.

4. Utilisation selon la revendication 1, **caractérisée en ce que** le cancer est le cancer du poumon.

5. Utilisation du ZD6474 ou d'un sel pharmaceutiquement acceptable de celui-ci et de la gemcitabine dans la fabrication d'un médicament destiné à être utilisé dans le traitement d'un cancer chez un animal à sang chaud, tel que l'homme, sous traitement avec des radiations ionisantes.

6. Utilisation selon la revendication 5, **caractérisée en ce que** le cancer implique une tumeur solide.

7. Utilisation selon la revendication 5, **caractérisée en ce que** le cancer est le cancer pancréatique.

8. Utilisation selon la revendication 5, **caractérisée en ce que** le cancer est le cancer du poumon.

9. Composition pharmaceutique, **caractérisée en ce qu'**elle comprend le ZD6474 ou un sel pharmaceutiquement acceptable de celui-ci et la gemcitabine, en association avec un excipient ou un support pharmaceutiquement acceptable.

10. Kit comprenant le ZD6474 ou un sel pharmaceutiquement acceptable de celui-ci et la gemcitabine.
